# EUROPEAN PATENT APPLICATION

(11) **EP 2 939 606 A1**
(43) Date of publication of application: **04.11.2015**
(21) Application number: 14166280.9
(22) Date of filing: 28.04.2014
(51) Int. Cl.: A61B 17/072

(54) **Surgical stapling apparatus**

(71) Applicant: Université de Liège, 4031 Angleur (BE); Centre Hospitalier Universitaire de Liège, 4000 Liège (BE)
(72) Inventor: De Roover, Arnaud, 4000 LIEGE (BE); Andersen, Bjarne, 3600 Frederikssund (DK); Videbaek, Karsten, 4040 Jyllinge (DK)

(57) **Abstract**

The present invention concerns a surgical stapling apparatus (100) comprising a first jaw (101) extending in a longitudinal direction (C) between a proximal end (101a) and a distal end (101b), a second jaw (102) facing said first jaw (101), a first hinge (103) joining said second jaw (102) to the distal end (101b) of the first jaw (101), and a first transmission device for actuating, from beyond the proximal end (101a) of the first jaw (101), a motion of the second jaw (102) with respect to the first jaw (101).

## Description

### TECHNICAL FIELD

The disclosure relates to a surgical stapling apparatus and more specifically to a surgical stapling apparatus comprising two opposing jaws hinged to each other.

### BACKGROUND

Surgical devices with opposing, hinged jaws for grasping tissue and joining it by surgical fasteners such as surgical staples are well known in the art. In some of these instruments a knife has also been provided for cutting through the joined tissue. Typically, one of the jaws carries at least one surgical staple, and usually a staple holder comprising a plurality of surgical staples arranged so as to be pushed against the opposite jaw by a wedge moving in a longitudinal direction, whereas the opposite jaw forms an anvil for plastically deforming the staple legs as they are driven against it. These staples can be arranged in at least two rows in said longitudinal direction and the knife may be arranged so as to cut the joined tissue longitudinally between these two rows. Such instruments have been disclosed, for example, in US Patents Nos. 3,079,606; 3,490,675; 4,429,695 and 5,065,929. These instruments were designed for use in conventional surgical procedures wherein surgeons have direct manual access to the tissue to be joined. However, in minimally invasive surgery, the tissue to be joined is accessed through a keyhole incision. Surgical stapling apparatuses adapted to such minimally invasive surgery have been disclosed for instance in US Patents Nos. 5,040,715; 5,307,976; 5,312,023; 5,318,221; 5,326,013; 5,332,142; and 5,865,361.

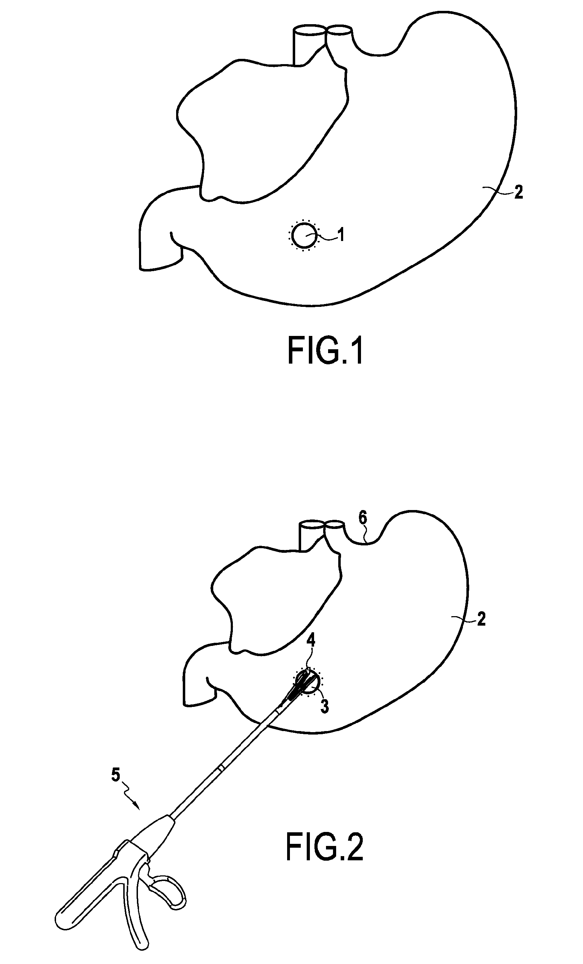

Each one of these surgical stapling apparatuses for minimally invasive surgery comprises a first jaw extending in a longitudinal direction between a proximal end and a distal end, a second jaw facing said first jaw, a hinge joining said second jaw to the proximal end of the first jaw, and means for actuating the closure of the second jaw against the first jaw from said proximal end. While this configuration facilitates the introduction of the jaws, in a closed state, into the patient's body through a keyhole incision, it limits the application of these devices to the stapling of tissue directly accessible from the side of the keyhole incision, which complicates some operations, even when the jaws can pivot together around a second hinge axis, as in the surgical stapling apparatus of US Patent No. 5,865,361. For instance, with such prior art surgical stapling apparatuses it is very difficult to start the stapling from the upper part of the stomach, that is, the fundus and pericardial area, which lie against the diaphragm in the left upper part of the abdominal cavity, since direct access is blocked by the ribcage and the diaphragm. Therefore, in order to perform a gastroplasty, whether on its own, or as part of an esophagus-lengthening procedure as in , for instance, the Collis-Nissen anti-reflux procedure, using such a surgical stapling apparatus through a keyhole incision in the abdomen, it has been proposed to first create, in a first step and using another, circular, stapler, a circular window 1 near the lesser curvature of the stomach 2, as seen on FIG. 1. One of the jaws 3,4 of the prior art surgical stapling apparatus 5 can then be inserted through this window 1 before closing the jaws 3,4 against each other to clamp and staple the stomach tissue between the window 1 and the His angle 6, as seen on FIG. 2.

Minimally invasive gastroplasty is, consequently, difficult to perform with prior art surgical stapling apparatuses, which has so far limited its uptake.

### SUMMARY

A first object of the disclosure is that of providing a surgical stapling apparatus allowing a reverse approach to the tissue to be stapled. For this purpose, in at least one illustrative embodiment, this surgical stapling apparatus comprises a first jaw extending in a longitudinal direction between a proximal end and a distal end, a second jaw facing said first jaw, a first hinge joining said second jaw to the distal end of the first jaw, and a first transmission device for actuating, from beyond the proximal end of the first jaw, a motion of the second jaw with respect to the first jaw.

Consequently, these jaws open in a direction opposite to those of prior art surgical stapling apparatuses, offering thus a reverse approach to the tissue to be stapled. Nevertheless, thanks to the first transmission device, the opening and or closure of the jaws can be actuated from beyond the proximal end of the first jaw, allowing thus the endoscopic operation of the jaws after their insertion, in the distal direction, through a keyhole incision in the patient's body.

This first transmission may comprise, for instance, a push-pull element, which provides a simple means for transmitting efforts in two opposite directions, thus allowing the actuation of both closing and opening movements of the second jaw through the same element. In particular, the push-pull element may be flexible, and the surgical stapling apparatus further comprise a channel extending in said longitudinal direction of the first jaw and a return guide around said first hinge said push-pull element, so that the flexible push-pull element is guided within this channel and return guide. The channel and return guide can prevent buckling of the flexible push-pull element when pushing, providing a particularly simple and thus reliable means of transmitting an actuation effort to the second jaw. This flexible push-pull element may in particular present a non-circular cross-section, and in particular a flattened cross-section, to further suppress buckling in at least one direction, while facilitating bending at the return guide. Also to facilitate bending at specific segments, the flexible push-pull element may present transversal notches. For strength, this flexible push-pull element may comprise one or more metallic cables for strength, possibly within a polymeric sheath to reduce friction. A substantially rigid push-pull element, like for instance a transmission rod, may however be used alternatively or in combination to this flexible push-pull element.

Furthermore, the first transmission device may further comprise a guide extending in said longitudinal direction of said first jaw, a slider engaging this guide and connected to said push-pull element of the first transmission device, and a sloping surface on the second jaw for engaging the slider. Consequently, the movement of the slider, driven through the flexible push-pull element, along the longitudinal direction of said first jaw and against the sloping surface on the second jaw, will reliably drive the closure of the second jaw against the first jaw. This slider may also comprise a knife with an edge oriented towards the proximal end of the first jaw, so as to cut any tissue clamped between the two jaws as the slider progresses towards the proximal end of the first jaw. To prevent accidentally cutting the tissue a second time after having performed the clamping and stapling operation, the surgical stapling apparatus may further comprise a catch for preventing movement of the slider in said longitudinal direction of the first jaw, towards said proximal end of the first jaw, beyond a predetermined threshold, after the slider has passed said predetermined threshold moving towards said proximal end in said longitudinal direction of the first jaw for a first time.

The surgical stapling apparatus may further comprise a wedge block configured to be actuated in a longitudinal direction of the second jaw by said slider, at least one surgical staple arranged within said second jaw so as to be pushed towards the first jaw by said wedge block moving in said longitudinal direction of the second jaw. In particular, the surgical stapling apparatus may comprise a plurality of surgical stapler arranged in at least one row and possibly at least two parallel rows, each row being oriented along said first longitudinal direction of the second jaw so that the travel of the wedge block, driven by the slider, along the longitudinal direction of the second jaw progressively pushes the staples of each row against the first jaw to staple the tissue clamped between the two jaws.

To facilitate operation of the surgical stapling apparatus, the second jaw may be biased towards an open position with respect to the first jaw, and in particular elastically biased towards said open position, although alternative biasing means, for instance magnetic means, can also be considered alternatively or in addition to elastic means such as a spring located between the jaws.

Furthermore, the surgical stapling apparatus may further comprise an elongated body and a second hinge, which connects a distal end of said elongated body to the proximal end of the first jaw. With this second hinge, which may have a hinge axis substantially orthogonal to both the longitudinal direction of the first jaw and a hinge axis of the first hinge, the first and second jaws can pivot together so as to better access the tissue to be stapled. Furthermore, to facilitate insertion of the jaws through a keyhole incision, this second hinge may be biased towards a centered position in which a longitudinal axis of the elongated body is substantially aligned with the longitudinal axis of the first jaw. To operate this second hinge after its insertion through a keyhole incision, the surgical stapling apparatus may also comprise a second transmission device for actuating, from beyond a proximal end of said elongated body, a pivoting movement of said first jaw with respect to said elongated body around a hinge axis of said second hinge. This second transmission device may also comprise a push-pull element, such as a transmission rod.

To facilitate handling of the surgical stapling apparatus, it may further comprise an interface for connecting a drive unit for driving at least said first transmission device, as well as a drive unit connected to said interface. This drive unit may comprise a handgrip and actuating elements such as a pushbutton and/or hand lever connected to said first and/or second transmission devices.

Another object of the disclosure is that of providing a surgical method for stapling difficult-to-access tissue. Accordingly, the present disclosure also relates to a use of such a surgical stapling apparatus, comprising the steps of introducing said jaws of the surgical stapling apparatus, with the distal end of the first jaw oriented forward, into a patient's body; positioning said jaws so that tissue to be stapled is located between the jaws in an open position, and actuating the closure of the jaws through said first transmission device to staple said tissue. In particular, the jaws of the surgical stapling apparatus may be introduced into the patient's body through the abdomen and said tissue to be stapled may be the stomach at the angle of His, to perform a gastroplasty, for instance. Furthermore, to allow minimally invasive surgery, wherein the step of introducing the jaws into the patient's body may be performed through a keyhole incision, the step of positioning the jaws be performed endoscopically and the step of actuating the jaws' closure be performed from outside the patient's body.

The above summary of some example embodiments is not intended to describe each disclosed embodiment or every implementation of the invention. In particular, selected features of any illustrative embodiment within this specification may be incorporated into an additional embodiment unless clearly stated to the contrary.

### BRIEF DESCRIPTION OF THE DRAWINGS

The invention may be more completely understood in consideration of the following detailed description of various embodiments in connection with the accompanying drawings, in which :
- FIG. 1 illustrates a first step in a gastroplasty performed with a prior art surgical stapling apparatus ;
- FIG. 2 illustrates a second step in the gastroplasty of FIG. 1 ;
- FIG. 3 illustrates a schematic perspective view of a surgical stapling apparatus according to a first embodiment;
- FIG. 4 is a detail view of a first transmission device of the surgical stapling apparatus of FIG. 1 ;
- FIG. 5 is a detail view of a first transmission device of a surgical stapling apparatus according to a second embodiment;
- FIG. 6 is a detail view of a first transmission device of a surgical stapling apparatus according to a third embodiment;
- FIG. 7 is a detail view of a first transmission device of a surgical stapling apparatus according to a fourth embodiment;
- FIG. 8 is a detail view of a first transmission device of a surgical stapling apparatus according to a fifth embodiment;
- FIG. 9 is a detail view of a first transmission device of a surgical stapling apparatus according to a sixth embodiment;
- FIG. 10 is an exploded view of a first and a second jaw of the surgical stapling apparatus of FIG. 3 ;
- FIGS. 11A to 11F are longitudinal cut views of an elongated body and pair of jaws of the surgical stapling apparatus of FIG. 3 in operation ;
- FIG. 12 is an exploded view of an elongated body of the surgical stapling apparatus of FIG. 3 ;
- FIGS. 13A to 13C are detail views of a hinge linking said elongated body to said first jaw in different positions ;
- FIGS. 14A and 14B are detail views of a lock for locking the first transmission device, in a locked and an unlocked position, respectively ;

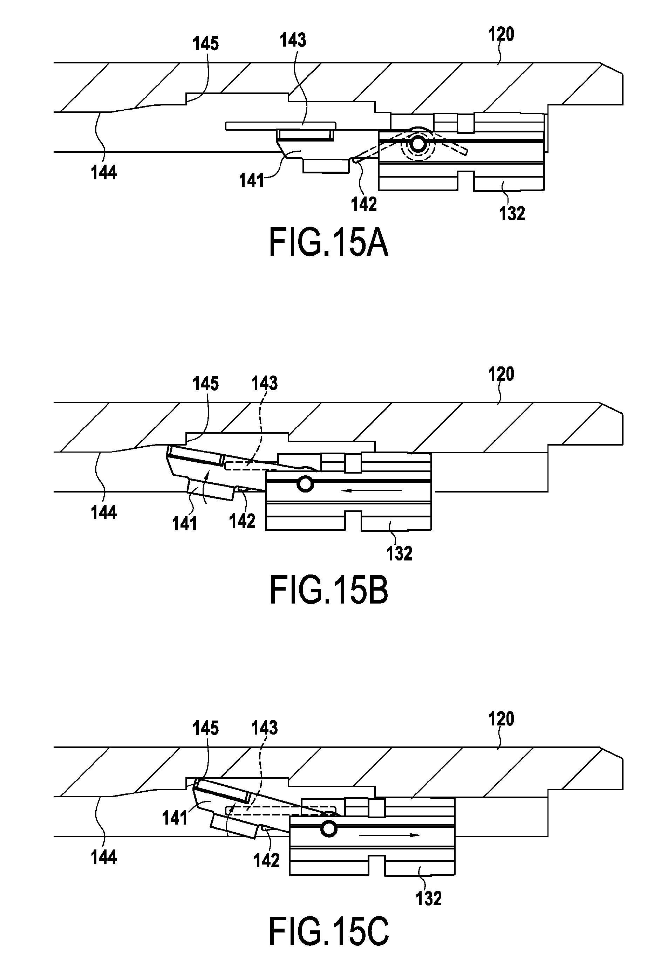

- FIGS. 15A to 15C are detail views of a catch for blocking the first transmission device, before a first actuation of the first transmission device, returning from said first actuation, and during a second actuation, respectively ; and
- FIGS. 16A to 16E show several steps in the use of the surgical stapling apparatus of FIG. 3 for performing a gastroplasty.

While the invention is amenable to various modifications and alternative forms, specifics thereof have been shown by way of example in the drawings and will be described in detail. It should be understood, however, that the intention is not to limit aspects of the invention to the particular embodiments described. On the contrary, the intention is to cover all modifications, equivalents, and alternatives falling within the scope of the invention.

### DETAILED DESCRIPTION

For the following defined terms, these definitions shall be applied, unless a different definition is given in the claims or elsewhere in this specification.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the invention. The illustrative embodiments depicted are intended only as exemplary. Selected features of any illustrative embodiment may be incorporated into an additional embodiment unless clearly stated to the contrary.

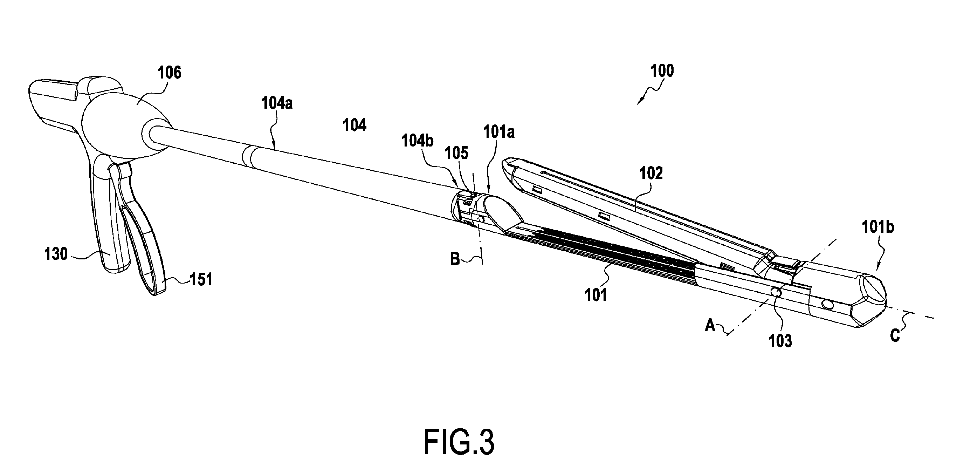

A surgical stapling apparatus 100 according to a first embodiment is illustrated on FIG. 3. This surgical stapling apparatus 100 comprises a first jaw 101 with a proximal end 101a and a distal end 101b; a second jaw 102, hinged to the distal end 101b of the first jaw 101 by a first hinge 103 with a first hinge axis A; an elongated body 104 with a distal end 104b hinged to the proximal end 101a of the first jaw 101 by a second hinge 105 with hinge axis B; and a drive unit 106 releasably connectable to a proximal end 104a of the elongated body 104. Hinge axis B of the second hinge 105 is substantially orthogonal to both hinge axis A of the first hinge 103 and a longitudinal axis C of the first jaw 101 extending between its proximal and distal ends 101a, 101b. To allow a remote actuation of a movement of the second jaw 102 towards and away from the first jaw 101, the surgical stapling apparatus 100 also comprises a first transmission device.

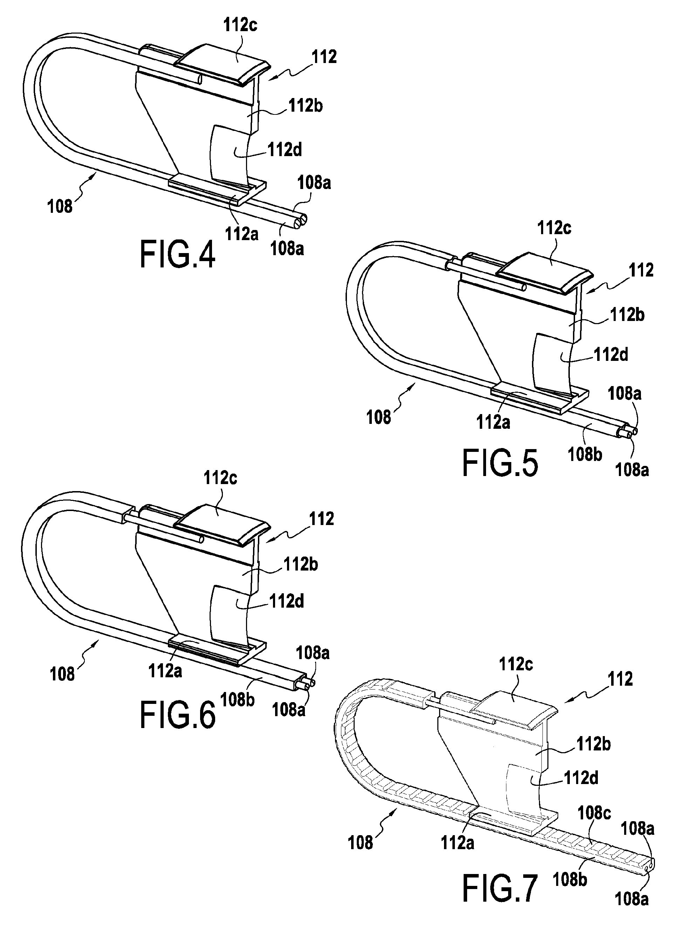

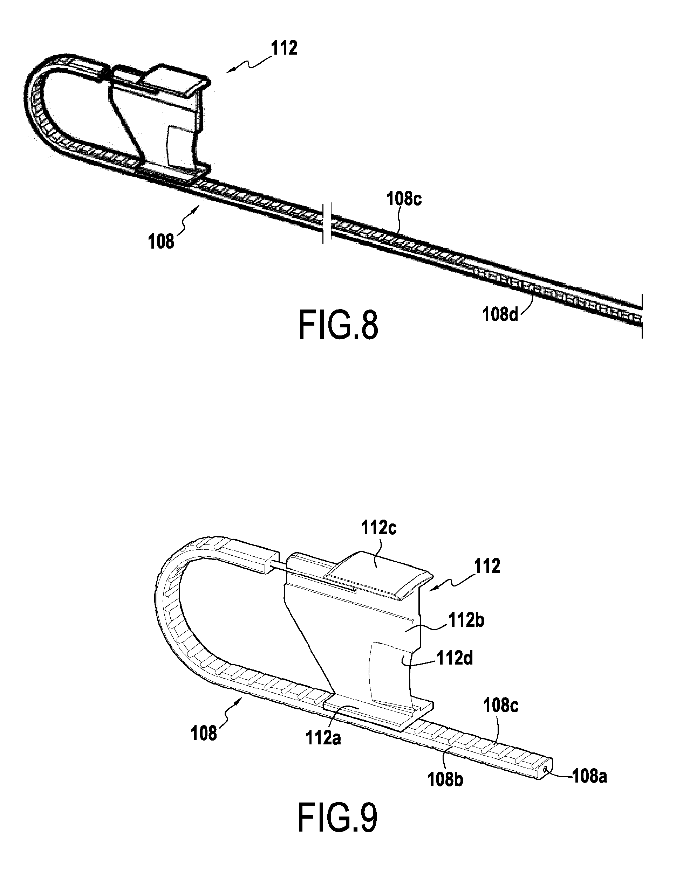

As shown in greater detail in FIG. 4, in this first embodiment the first transmission device comprises a flexible push-pull element 108 in the form of a pair of cables 108a arranged side-by-side. In the illustrated embodiment, these cables 108a are metallic and have a round cross-section each. They may in particular be in surgical stainless steel, although alternative materials and cross sections can also be considered alternatively or in combination with these. In alternative embodiments shown in FIGS. 5 to 9, these cables 108a may be sheathed in a polymeric sheath 108b, for example of a fluoropolymeric material such as polytetrafluoroethylene or perfluoroalkoxy, so as to reduce friction on the flexible push-pull element 108. In the alternative embodiment shown on FIG. 5, both cables 108a are within a single polymeric sheath 108b with a bilobate cross-section forming a figure-of-eight shape around the two cables 108a. In the alternative embodiment shown in FIG. 6, the cross-section of the sheath 108b is rectangular and flattened to facilitate bending the flexible push-pull element 108 around the first hinge 103. To further facilitate this bending, in the alternative embodiment shown in FIG. 7, upper and lower surfaces of the sheath 108b present transversal notches 108c on at least a longitudinal segment of the flexible push-pull element 108. In the alternative embodiment shown in FIG. 8, in order to also facilitate bending at the second hinge 105, lateral surfaces of the sheath 108b also present transversal notches 108d on another longitudinal segment of the flexible push-pull element 108. Finally, in the alternative embodiment shown in FIG. 9, the flexible push-pull element 108 comprises a single cable 108a within the sheath 108b. Each one of these alternative flexible push-pull elements 108 can be used alternatively to the unsheathed cables of the first embodiment in otherwise equivalent surgical stapling apparatuses.

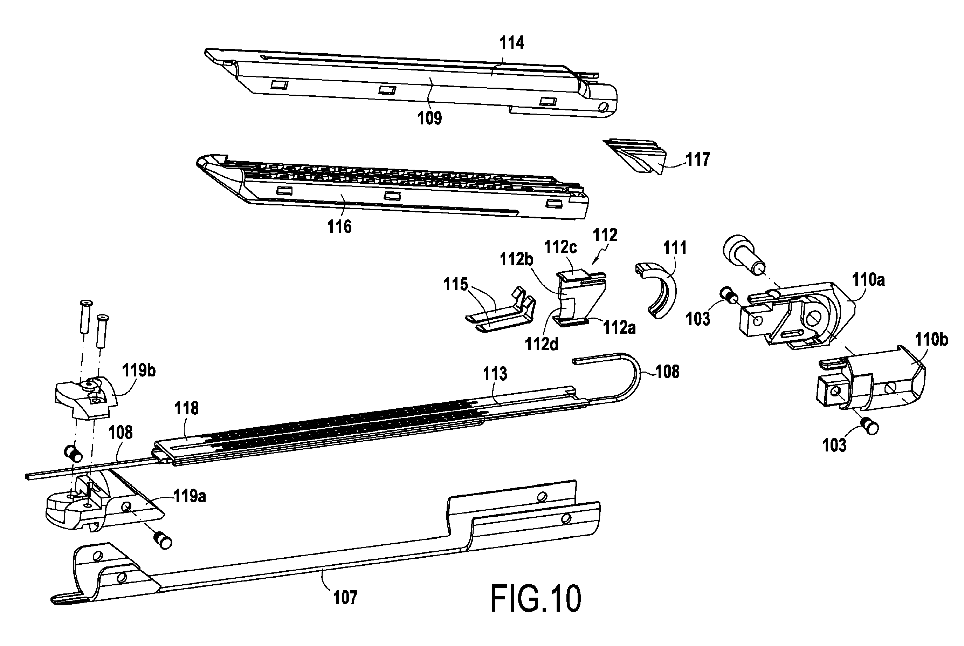

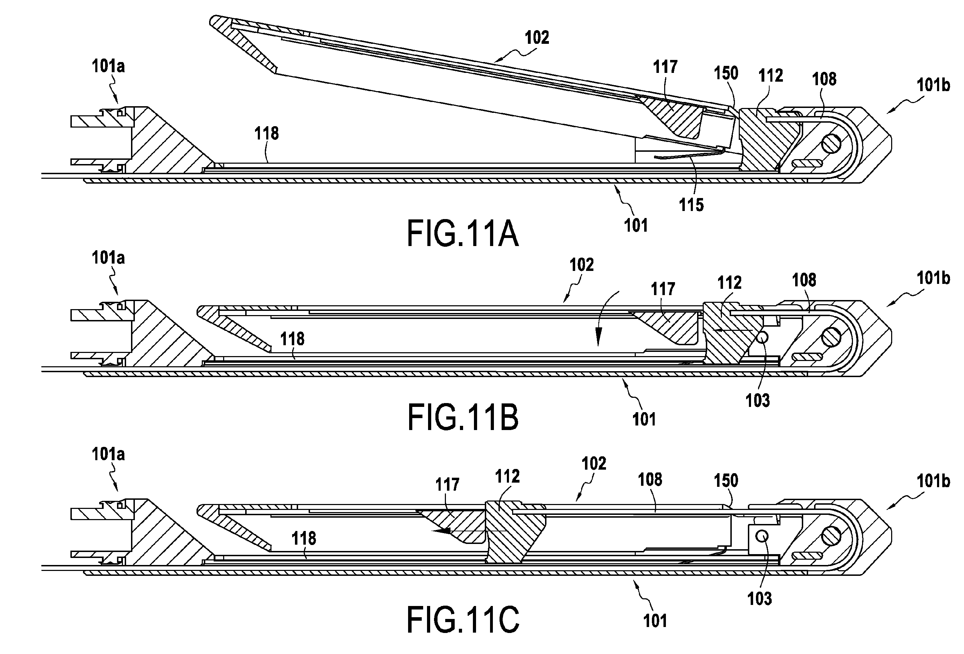

Turning now to FIGS. 10 and 11A to 11F, the flexible push-pull element 108 is guided within a longitudinal channel in the first jaw 101, a return guide 111 around the first hinge 103 at the distal end 101b of the first jaw 101, and a longitudinal channel in the second jaw 102, so that this flexible push-pull element 108 can transmit a push effort without buckling, while bending around at least the first hinge 103. In the second jaw 102, one end of this flexible push-pull element 108 is fixed to a slider 112. A lower flange 112a of this slider 112 is retained within a longitudinal guide 113 in the first jaw 101, whereas an upper end 112b traverses a longitudinal slit 114 through the second jaw 102, and culminates in an upper flange 112c retained against an opposite side of the second jaw 102. Both flanges 112a, 112c may present low-friction inserts (not shown) to reduce their friction against contact surfaces in the first and second jaws 101, 102. Between the flanges 112a, 112c, the slider 112 further comprises a knife 112d with its edge oriented towards the proximal end 101a of the first jaw 101.

The first jaw 101 comprises a lower cover 107, an anvil 118 mounted on this lower cover 107, a base part 119 and a cover part 120 at the proximal end 101a, and two half-shells 110a, 110b holding between them both the return guide 111 and the first hinge 103 at the distal end 101a. Springs 115 located between the jaws 101, 102, close to the first hinge 103, bias the jaws 101, 102 towards an open position. The second jaw 102 comprises an upper cover 109 and a staple holder 116, holding a plurality of surgical staples arranged in longitudinal rows to each side of the longitudinal slit 114. Pushers (not shown) aligned with these staples protrude from the upper side of the staple holder 116 and into a longitudinal cavity of the second jaw 102. This longitudinal cavity, formed between the staple holder 116 and the cover 109 of the second jaw 102, also contains a wedge block 117, engageable by the slider 112, and configured so as to push, in a longitudinal movement towards the proximal end 101a of the first jaw 101, said pushers and staples transversally towards an anvil 118 integrated in the first jaw 101, as shown with reference to FIGS. 11A to 11F.

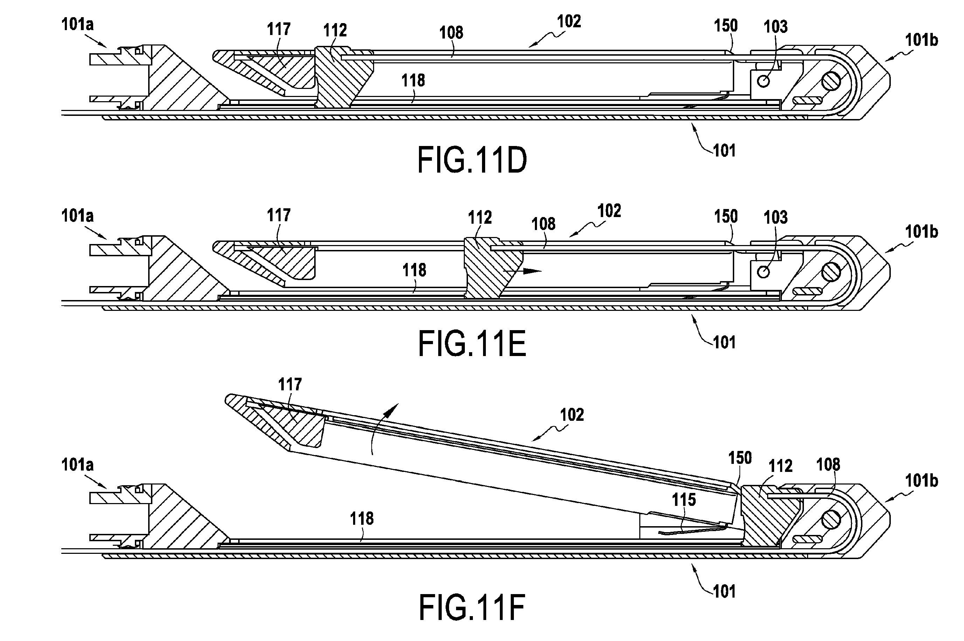

In FIG. 11A, the jaws 101,102 are held open in an open position by the elastic springs 115, and the slider 112 is located near the distal end 101b of the first jaw 101. Starting from this initial open position, the slider 112 can be pushed by the flexible push-pull element 108 towards the proximal end 101a of the first jaw 101, guided in the longitudinal direction C of the first jaw 101 by the longitudinal guide 113 in which its lower flange 112a is engaged. In this motion, the upper flange 112c of the slider 112 will first engage a sloped surface 150 on the upper side of the second jaw 102, closing the second jaw 102 against the first jaw 101, as shown in FIG. 11B, and thus clamping any tissue held between them. The slider 112 can then continue to be pushed by the flexible push-pull element 108 towards the proximal end 101a of the first jaw 101, as shown on FIG. 11C. During this travel, the upper flange 112c, pressing against the upper side of the second jaw 102, holds it against the first jaw 101, while the slider 112 pushes the wedge block 117 before it. As this wedge block 117 advances towards the proximal end 101a of the first jaw 101, it engages successive pushers protruding from the staple holder 116, pushing them and the corresponding staples against the anvil 118 of the first jaw 101. The legs of the staples are folded inwards by their impact against the anvil 118, thus stapling any tissue clamped between the jaws 101, 102. Behind the wedge block 117, the knife 112d cuts through the clamped tissue between the staples. This movement can proceed until the opposite end of the second jaw 102, as shown on FIG. 11D, although it may be interrupted before. After stapling and cutting as far as desired within the length of the second jaw 102, the movement can be reversed by pulling back the slider 112 with the flexible push-pull element 108, as shown on FIG. 11E. As shown in this drawing, the wedge block 117 will not necessarily follow the slider 112 in its return. As the slider 112 arrives again at the distal end 101b of the first jaw 101, retreating behind the sloped surface 150, the springs 115 will open the jaws 101, 102 again, as shown on FIG. 11F.

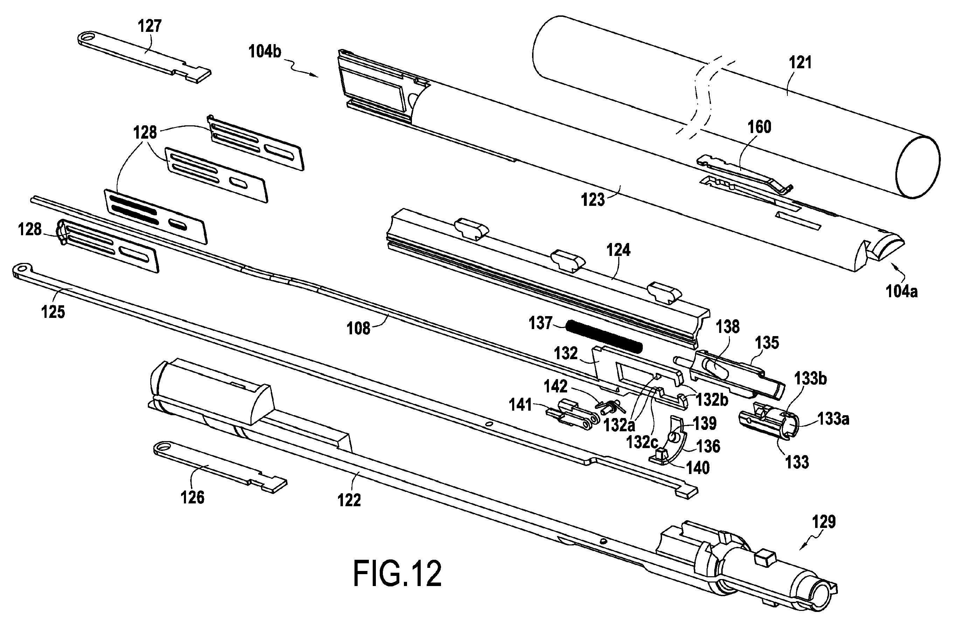

The elongated body 104 is shown in greater detail in FIG. 12. This elongated body 104 comprises an outer sleeve 121 and, within this outer sleeve 121, a base part 122 and a cover part 123 linked by a side support 124. It also presents a longitudinal channel for guiding the flexible push-pull element 108 between the proximal and distal ends 104a, 104b of the elongated body. In this particular embodiment, this longitudinal channel is delimited by the base part 122 and the outer sleeve 121.

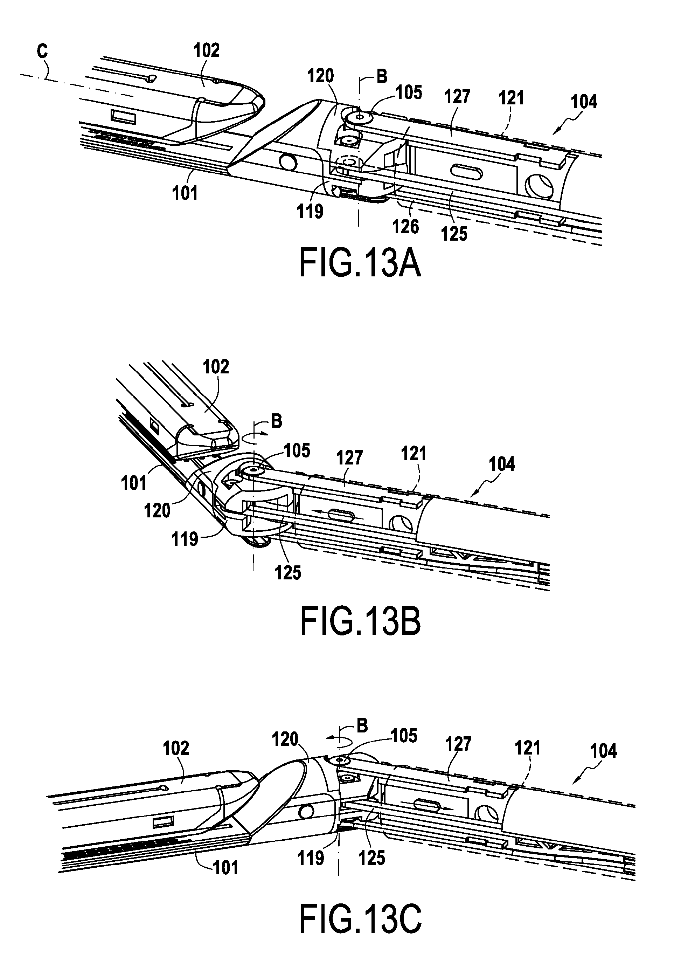

To provide a remote actuation of a pivoting motion of the first and second jaws 101, 102 with respect to this elongated body 104, the surgical stapling apparatus 100 also comprises a second transmission device including a transmission rod 125 received within another longitudinal channel in the elongated body 104. As seen on FIGS. 13A to 13C, this transmission rod 125 is laterally offset with respect to the hinge axis B of the second hinge 105 and pivotally linked to the proximal end 101a of the first jaw 101, between the base part 119 and cover part 120 forming the proximal end 101a of the first jaw 101 in this particular embodiment.

The second hinge 105 is held between two hinge brackets 126, 127, which are held in recesses in the elongated body 104 and protrude from these in a distal direction. The distal end 104b of the elongated body 104 and the proximal end 101a of the first jaw 101 are also linked by leaf springs 128 arranged to as to return the second hinge 105 towards the neutral position illustrated in FIG. 13B in which the longitudinal axis C of the first jaw 101 is substantially aligned with a longitudinal axis of the elongated body 104. Pushing the transmission rod 125 will pivot the first and second jaws 101, 102 relative to the elongated body 104 in a first direction around the hinge axis B of the second hinge 105, as shown in FIG. 12B, whereas pulling the transmission rod 122 will pivot the first and second jaws 101, 102 in an opposite direction, as shown in FIG. 12C.

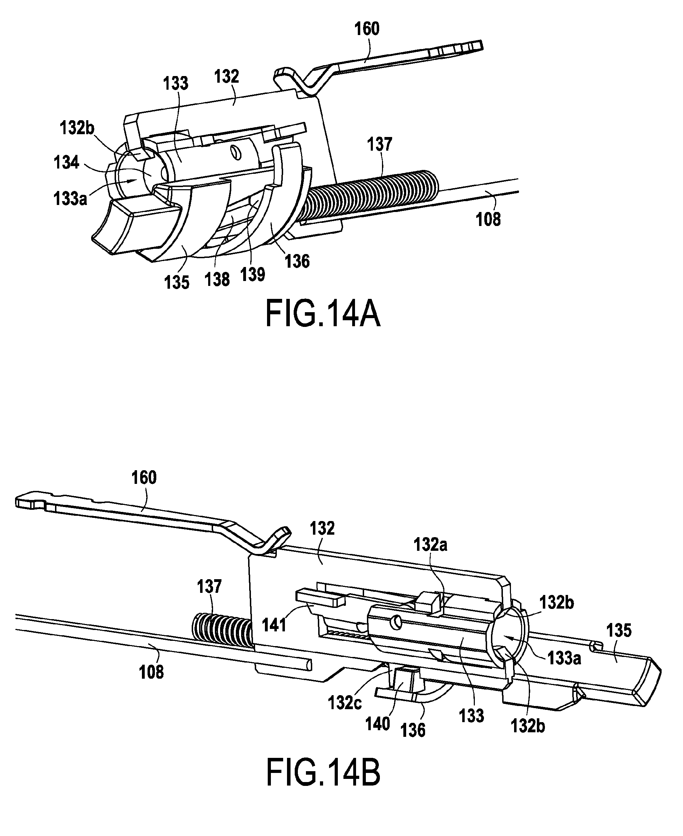

Going back to FIG. 12, the elongated body 104 comprises an interface 129 at its proximal end 104a for releasably connecting the elongated body 104 to the drive unit 106. As previously shown in FIG. 3, this drive unit 106 comprises, besides a handgrip 131, actuating elements, such as the lever 151, for actuating the first and second transmission devices. The first transmission device also comprises a plate 132, fixed to the flexible push-pull element 108, by laser welding for example, and a connector 133, received within a cutout in said plate 132 in driving engagement with two guide dowels 132a protruding into said cutout, as shown in FIG. 14A. This connector 133 presents a central recess 133a for receiving a connecting rod (not illustrated) coupled to the lever 130 of the drive unit. An inner pressure wall 134 for receiving a pushing effort from the connecting rod closes this recess 133a. Two retraction hooks 134 of the plate 131 protrude, through radial cutouts 133b in the connector 133, into its central recess 133a. These retraction hooks 132b are configured to receive a pulling effort from the connecting rod of the drive unit 106 when engaged into the central recess 133a of the connector 133. An indexing spring 160 is held in a recess in the cover part 123, protruding inwards to engage a step in the plate 132 to index a predetermined default position of the first transmission device.

The surgical stapling apparatus 100 also comprises, as shown in particular in FIGS. 14A to 14D, a lock for locking the first transmission device when the drive unit 106 is disconnected from the elongated body 104. This lock comprises an activation slider 135, a locking block 136 and a return spring 137. The activation slider 135 comprises a helical cam guide 138, and the locking block 136 a follower 139 in engagement with this cam guide 138, as well as a locking pin 140. When the drive unit 106 is not connected to the elongated body 104, the return spring 137 holds the activation slider 135 and locking block 136 in a first position, illustrated in FIGS. 14A and 14B, in which this activation slider 135 protrudes at the proximal end 104a of the proximal body 104. In this first position, the locking pin 140 engages a notch 132c in the plate 132, positively locking it in a longitudinal direction.

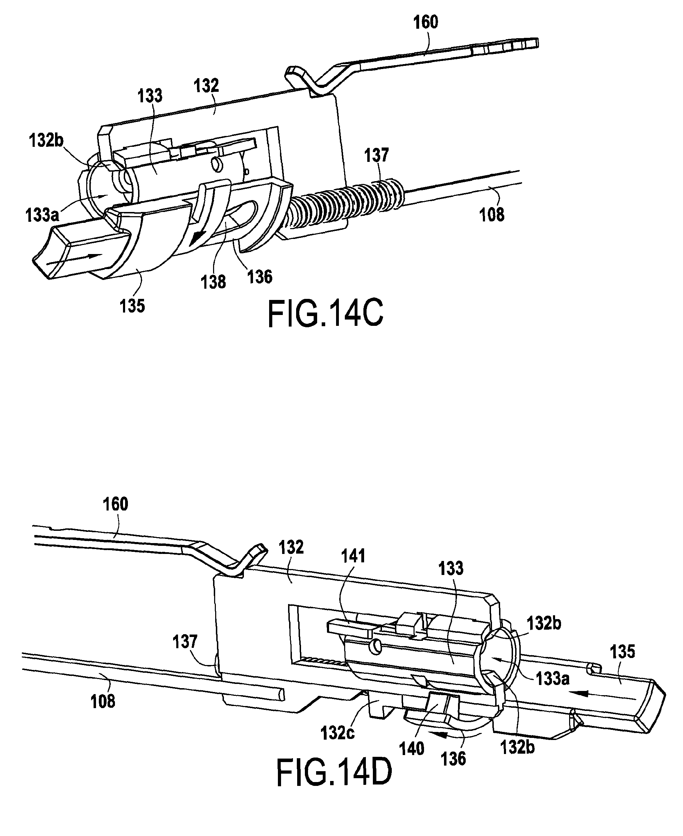

When the drive unit 106 is connected to the elongated body 104, it pushes the activation slider 137, against the return spring 139, towards a second position illustrated in FIGS. 14C and 14D. In this movement, through the engagement of its cam guide 138 with the follower 139, the activation slider 135 rotates the locking block 136 out of engagement with the plate 132, unlocking the first transmission device. If the drive unit 106 is disconnected from the elongated body 104, the return spring 139 will bring the activation slider 137 back towards its first position, locking again the first transmission device.

After having used the surgical stapling apparatus 100 a first time to clamp, staple and cut some tissue, a surgeon may also want to prevent a second, accidental actuation of the first transmission device at least beyond a predetermined threshold, in particular to prevent that the knife 112d cuts again through the tissue. For this purpose, the surgical stapling apparatus 100 of the present embodiment also includes a latch 141 hinged to the connector 133, and biased by a torsion spring 142 towards a locking position, a rib 143 protruding from the cover part 120 of the elongated body 104, and a slide surface 144 with a step 145 in the same cover part 120. Initially, as seen on FIG. 14A, the rib 143 holds the latch 141 in a first position with respect to the connector 133, in which the connector 133 can move freely in the longitudinal direction. When the first transmission device reaches a first predetermined position, the latch 141 reaches a distal end of the rib 143 and is forced by the torsion spring 142 towards a second position with respect to the connector 133, in contact with the slide surface 144, as seen on FIG. 14B. The latch 141 can then continue its travel in a distal direction on said slide surface 144. However, when the first transmission device is pulled back, the latch 141 reaches the step 145 where, no longer supported by the slide surface 144, it is rotated by the torsion spring 142 towards a third position with respect to the connector 132, as shown on FIG. 14C. In this third position, the latch 141 will face the step 145 and abut against it when moving again in the distal direction. The movement of the latch 141 and the connector 132 beyond this step 145 is thus blocked. The position of the step 145 may for example be such that it still allows movement of the first transmission device between the position corresponding to FIG. 10A, in which the jaws 101, 102 are open, and the position corresponding to FIG. 10B, in which the jaws 101, 102 have just closed, but not beyond.

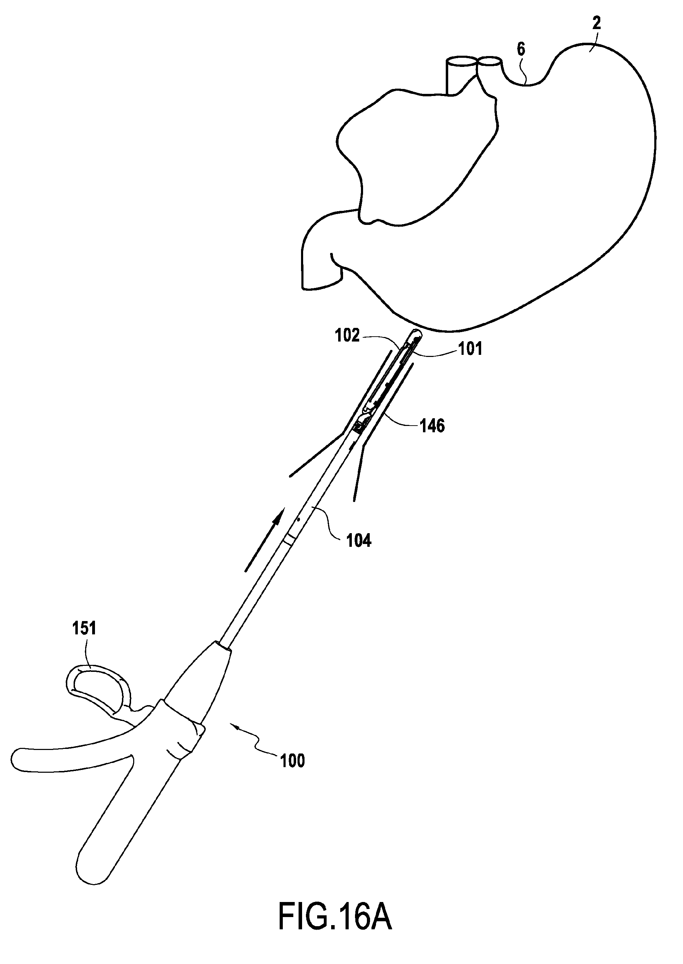

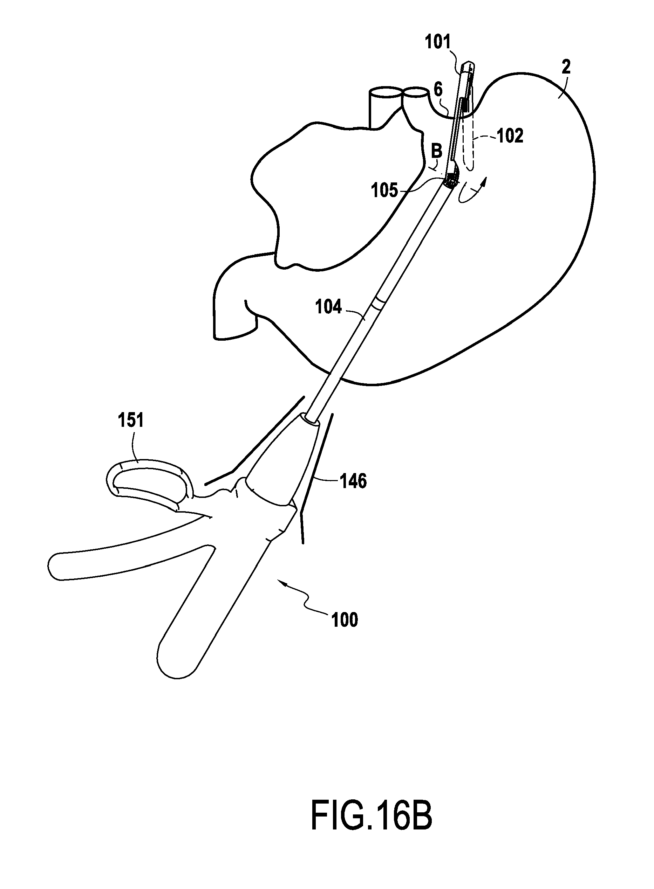

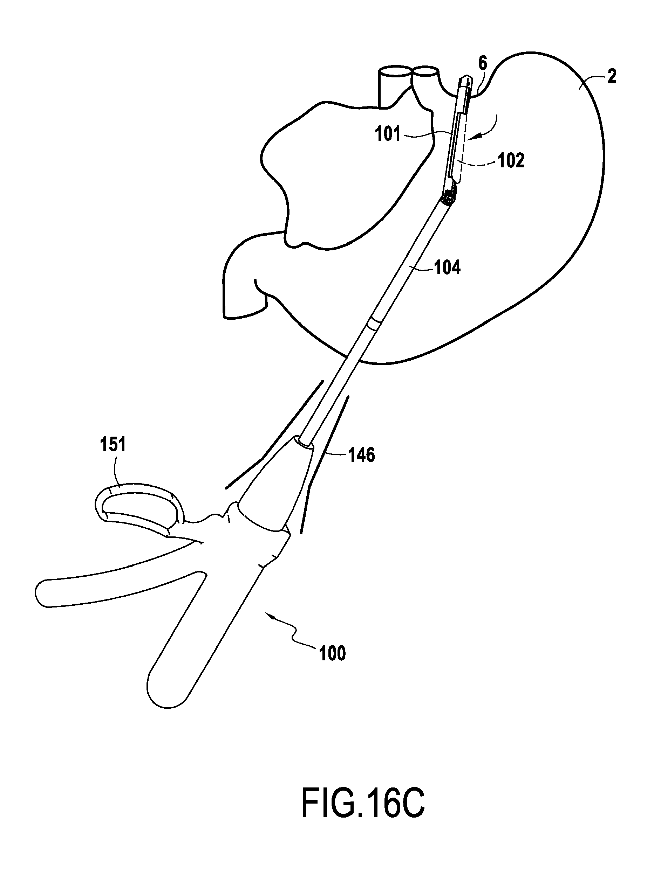

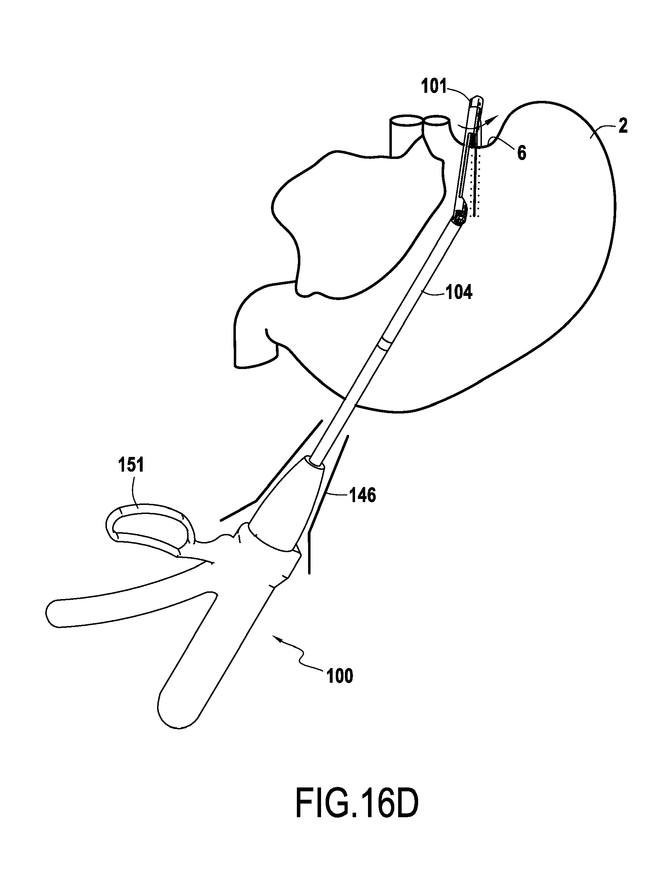

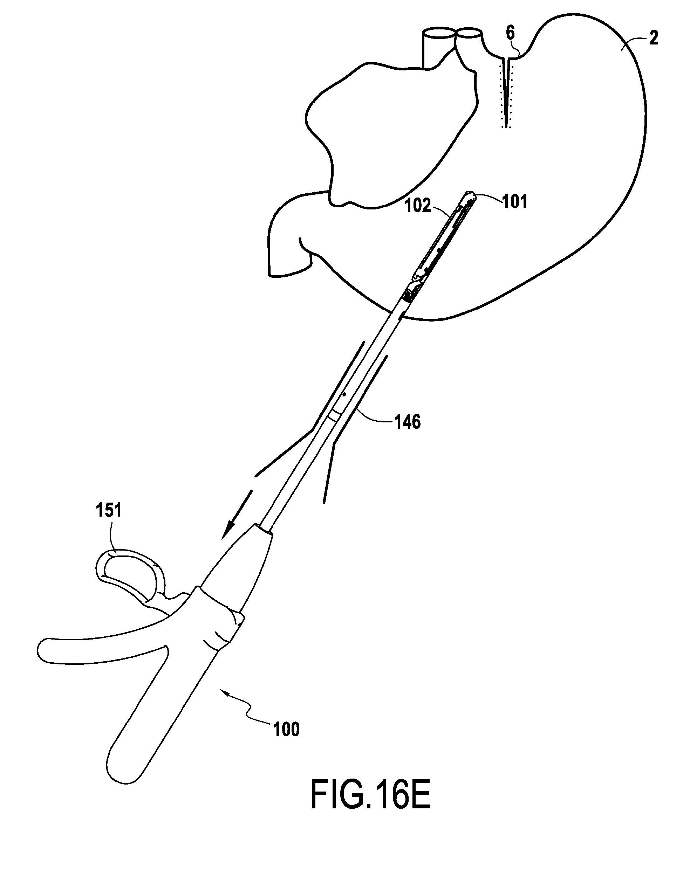

A use of the surgical stapling apparatus 100 for performing a gastroplasty is shown in FIGS. 156 to 16E. In a first step, shown in FIG. 16A, the jaws 101, 102 and elongated body 104 are inserted, with the distal end 101b of the first jaw oriented forward, through a trocar 146 placed in a keyhole incision in the patient's abdomen. Once inserted, the jaws 101, 102 are sprung open by the springs 115, and the jaws 101, 102 are positioned, with endoscopic guidance, about the His angle 6 of the stomach 2, as shown in FIG. 16B. In this second step, to better position the jaws 101, 102 with respect to the His angle 6, they may be pivoted around the hinge axis B of the second hinge 105 with respect to the elongated body 104. This movement may be actuated, through the second transmission device, from the drive unit 106 outside of the patient's body using the second transmission device. In the next step, shown in FIG. 16C, the closure of the second jaw 102 against the first jaw 101 is also actuated from the drive unit 106 outside the patient's body, using the first transmission device. The jaws 101, 102 clamp the stomach, and further movement of the lever 151, acting on the first transmission device, drives the slider 112 towards the proximal end 101a of the first jaw 101, stapling the walls of the stomach 2 and cutting with the knife 112d between the rows of staples. After finishing this operation, the jaws 101, 102 can be opened again to unclamp the stomach 2, as seen in FIG. 16D, and closed again to retreat them, through the trocar 146, out of the patient's body, as seen in FIG. 16E. Such a gastroplasty may be performed within a more complex operation, such as, for instance, Vertical Banded Gastroplasty, Collis-Nissen Fundoplication, or Magenstrasse and Mill Gastroplasty.

Those skilled in the art will recognize that the present invention may be manifested in a variety of forms other than the specific embodiments described and contemplated herein. For instance, assemblies comprising pairs of jaws and/or elongated bodies of various shapes and lengths may be provided for connection with a single drive unit, so that the same drive unit may be used to complete different surgical stapling apparatuses for use in different procedures and/or different patients. Accordingly, departure in form and detail may be made without departing from the scope of the present invention as described in the appended claims.

## Claims

1. Surgical stapling apparatus (100) comprising :
a first jaw (101) extending in a longitudinal direction (C) between a proximal end (101a) and a distal end (101b) ;
a second jaw (102) facing said first jaw (101) ;
a first hinge (103) joining said second jaw (102) to the distal end (101b) of the first jaw (101) ; and
a first transmission device for actuating, from beyond the proximal end (101a) of the first jaw (101), a motion of the second jaw (102) with respect to the first jaw (101).

2. Surgical stapling apparatus (100) according to claim 1, wherein said first transmission device comprises a push-pull element (108).

3. Surgical stapling apparatus (100) according to claim 2, further comprising :
a channel extending in said longitudinal direction (C) of the first jaw (101) ;
a return guide (111) around said first hinge (103) ;
wherein said push-pull element (108) of the first transmission device is flexible and guided within this channel and return guide (111).

4. Surgical stapling apparatus (100) according to claim 3, wherein said first transmission device further comprises :
a guide (113) extending in said longitudinal direction of said first jaw (101);
a slider (112) engaging this guide (113) and connected to said push-pull element (108) of the first transmission device ; and
a sloping surface (150) on the second jaw (102) for engaging the slider (112).

5. Surgical stapling apparatus (100) according to claim 4, wherein said slider (112) further comprises a knife (112d) with an edge oriented towards the proximal end (101a) of the first jaw (101).

6. Surgical stapling apparatus (100) according to claim 5, further comprising a latch (141) for preventing movement of the slider (112) in said longitudinal direction (C) of the first jaw (101), towards said proximal end (101a) of the first jaw (101), beyond a predetermined threshold, after the slider (112) has passed said predetermined threshold moving towards said proximal end (101a) of the first jaw (101) for a first time.

7. Surgical stapling apparatus (100) according to any one of claims 4 to 6, further comprising :
a wedge block (117) configured to be actuated in a longitudinal direction of the second jaw (102) by said slider (112) ; and
at least one surgical staple arranged within said second jaw (102) so as to be pushed towards the first jaw (101) by said wedge block (117) moving in said longitudinal direction of the second jaw (102).

8. Surgical stapling apparatus (100) according to any one of the preceding claims, wherein the second jaw (102) is biased towards an open position with respect to the first jaw (101).

9. Surgical stapling apparatus (100) according to any one of the preceding claims, further comprising an elongated body (104) and a second hinge (104) connecting a distal end (104b) of said elongated body to the proximal end (101a) of the first jaw (101).

10. Surgical stapling apparatus (100) according to claim 9, wherein said second hinge (104) has a hinge axis (B) substantially orthogonal to both the longitudinal direction (C) of the first jaw (101) and a hinge axis (A) of the first hinge (103).

11. Surgical stapling apparatus (100) according to any one of claims 9 or 10, wherein said second hinge (105) is biased towards a centered position in which a longitudinal axis of the elongated body (104) is substantially aligned with the longitudinal axis (C) of the first jaw (101).

12. Surgical stapling apparatus (100) according to any one of claims 9 to 11, further comprising a second transmission device for actuating, from beyond a proximal end (104a) of said elongated body (104), a pivoting movement of said first jaw (101), with respect to said elongated body (104), around a hinge axis (B) of said second hinge (105).

13. Surgical stapling apparatus (100) according to claim 12, wherein said second transmission device comprises a push-pull element (125).

14. Surgical stapling apparatus (100) according to any one of the previous claims, further comprising an interface (129) for connecting a drive unit (106) for driving at least said first transmission device.

15. Surgical stapling apparatus (100) according to claim 14, further comprising a drive unit (106) connected to said interface (129) for driving at least said first transmission device.

16. Use of a surgical stapling apparatus (100) according to any one of the previous claims, comprising the steps of:
introducing said jaws (101,102) of the surgical stapling apparatus (100), with the distal end (101b) of the first jaw (100) oriented forward, into a patient's body ;
positioning said jaws (101,102) so that tissue to be stapled is located between the jaws (101,102) ; and
actuating, through said first transmission device, a closure of said jaws (101,102) to clamp and staple said tissue.

17. Use of a surgical stapling apparatus (100) according to claim 16, wherein the jaws (101,102) of the surgical stapling apparatus (100) are introduced into the patient's body through the abdomen and said tissue to be stapled is the stomach (2) at the angle of His (6).

18. Use of a surgical stapling apparatus (100) according to any one of claims 16 or 17, wherein the step of introducing the jaws (101,102) into the patient's body is performed through a keyhole incision, the step of positioning the jaws (101,102) is performed with endoscopic guidance and the step of actuating the closure of the jaws (101,102) is performed from outside the patient's body.
